(19) Europäisches Patentamt / European Patent Office / Office européen des brevets

(11) **EP 1 752 535 A1**

(12) **EUROPEAN PATENT APPLICATION**
published in accordance with Art. 158(3) EPC

(43) Date of publication:
**14.02.2007 Bulletin 2007/07**

(51) Int Cl.:
***C12N 15/09*** $^{(2006.01)}$

(21) Application number: **05743478.9**

(86) International application number:
**PCT/JP2005/009885**

(22) Date of filing: **30.05.2005**

(87) International publication number:
**WO 2005/116208 (08.12.2005 Gazette 2005/49)**

(84) Designated Contracting States:
**DE FR GB**

(30) Priority: **31.05.2004 JP 2004161879**

(71) Applicant: **Nichirei Foods Inc.**
**Tokyo 104-8402 (JP)**

(72) Inventors:
• **Koizumi, Takeshi,**
**c/o Nichirei Corporation**
**Mihama-ku,**
**Chiba-shi,**
**Chiba 2618545 (JP)**

• **Yamamoto, Satoshi,**
**c/o Nichirei Corporation**
**Mihama-ku,**
**Chiba-shi,**
**Chiba 2618545 (JP)**

(74) Representative: **Desaix, Anne**
**Ernest Gutmann - Yves Plasseraud S.A.S.**
**3, rue Auber**
**75009 Paris (FR)**

(54) **DNA AMPLIFICATION METHOD**

(57) The object of the invention is to provide a DNA amplification method with which single-strand DNA of a desired direction and a desired region can be prepared simply and efficiently. The invention provides a DNA amplification method that uses DNA as a template, wherein the DNA amplification method uses a first primer that is complementary to the 3' end side of a target base sequence, a second primer that has a base sequence that is homologous to the 5' end side of that target base sequence, and a third primer that has, on its 3' end side, a base sequence that is homologous to part of the base sequence on the 5' end side of the second primer or the first primer, and a melting temperature $Tm_1$ of the first primer, a melting temperature $Tm_2$ of the second primer, and a melting temperature $Tm_3$ of the third primer have the relationship expressed by the following expression:

$$Tm_1 < Tm_3 \quad \text{and} \quad Tm_2 < Tm_3$$

and
wherein the DNA amplification method includes a first process of performing a PCR cycle at an annealing temperature Ta (where $Ta \leq Tm_1$ and $Ta \leq Tm_2$), and a second process of performing a PCR cycle at an annealing temperature Tb (where $Tm_1 < Tb$, $Tm_2 < Tb$, and $Tb \leq Tm_3$).

EP 1 752 535 A1

SEQUENCE LISTING

<110> NICHIREI FOODS Inc.

<120> DNA AMPLIFICATION METHOD

<130> B6994 - JAZ/KN(SDU)

<140> EP 05743478.9
<141> 2005-05-30

<150> JP 20040161879
<151> 2004-05-31

<150> PCT/JP2005/009885
<151> 2005-05-30

<160> 4

<170> PatentIn version 3.3

<210> 1
<211> 135
<212> DNA
<213> Vibrio parahaemolyticus

<400> 1
tgctgtgttc gtaaaatcag atcaagtaca gcttcaacat tcctatgatt ctgtagctaa       60

ctttgttggt gaagatgaag gttctattcc aagtaaaatg tatttggatg aaactccaga      120

atattttgtt aatgt      135


<210> 2
<211> 20
<212> DNA
<213> Artificial

<220>
<223> Synthetic polynucleotide which works as a reverse primer on the
      1st step (PCR cycle I.)

<400> 2
acattaacaa aatattctgg      20


<210> 3
<211> 18
<212> DNA
<213> Artificial

<220>
<223> Synthetic polynucleotide containing 10 bases of the same sequence
      on its 5'-end as the nucleotide represented by the sequence list
      numbered 4, which works as a forward primer solely on the 1st
      step (PCR cycle I.)

<400> 3
tgctgtgttc gtaaaatc      18

```
<210>  4
<211>  18
<212>  DNA
<213>  Artificial

<220>
<223>  Synthetic polynucleotide containing the same sequence on its 3'
       -end as the nucleotide represented by the sequence list numbered
       3, which works as a forward primer both on the 1st step (PCR
       cycle I.) and the 2nd step (PCR cycle II.)

<400>  4
gggcgggctg ctgtgttc                                                   18
```

SEQUENCE LISTING

<110> NICHIREI FOODS CORPORATION

<120> Method for DNA amplification

<130> PC9611

<160> 4

<210> 1

<211> 135

<212> DNA

<213> Vibrio parahaemolyticus

<400> 1

tgctgtgttc gtaaaatcag atcaagtaca gcttcaacat tcctatgatt ctgtagctaa 60

ctttgttggt gaagatgaag gttctattcc aagtaaaatg tatttggatg aaactccaga 120

atattttgtt aatgt 135

<210> 2

<211> 20

<212> DNA

<213> Artificial Sequence

<220>

<223> Synthetic polynucleotide which works as a reverse primer on the 1st step ( PCR cycle I.)

<400> 2

acattaacaa aatattctgg 20

<210> 3

<211> 18

<212> DNA

<213> Artificial Sequence

<220>

<223> Synthetic polynucleotide containing 10 bases of the same sequence on its 5'-end as the nucleotide represented by the sequence list numbered 4, which works as a forward primer solely on the 1st step(PCR cycle I.)

<400> 3

tgctgtgttc gtaaaatc 18

<210> 4

<211> 18

<212> DNA

<213> Artificial Sequence

<220>

<223> Synthetic polynucleotide containing the same sequence on its 3'-end as the nucleotide represented by the sequence list numbered 3, which works as a forward primer both on the 1st step(PCR cycle I) and the 2nd step(PCR cycle II.)

<400> 4

gggcgggctg ctgtgttc 18

**Description**

BACKGROUND OF THE INVENTION

Field of the invention

The present invention relates to methods for amplifying DNA.

Description of the Related Art

To identify microorganisms in medical, public health, and food sanitation fields, and in genetic analysis for clinical diagnoses, for example, a single strand of DNA is prepared from double-strand DNA originating from the specimen and used for base sequence determination (sequencing). To detect DNA having a specific sequence, there is the technique of using single-strand DNA obtained through DNA amplification in which a minute amount of specimen DNA serves as a template.

Single-strand DNA generally is prepared by asymmetrical PCR (polymerase chain reaction).

Conventional asymmetrical PCR methods that have been proposed include a method of biasing the concentration of the forward primer and the reverse primer (for example, see Non-Patent Document 1), a method of exploiting the fact that RNA primers are not as easily amplified as DNA primers by using a DNA/RNA hybrid primer and taking advantage of the difference in amplification ability between primers (for example, see Non-Patent Document 2), a so-called thermal asymmetric method that utilizes the difference in melting temperatures (Tm) of the primers (for example, see Non-Patent Document 3), and a method of using a blocker for the DNA extension reaction to inhibit the extension of one DNA strand of a double-strand DNA template (for example, see Patent Documents 1 and 2).

Patent Document 1: United States Patent No. 5849497
Patent Document 2 : United States Patent No. 5627054
Non-Patent Document 1: Proceedings of the National Academy of Sciences, 85 (1988), p.7652-7656
Non-Patent Document 2 : Nucleic Acids Researech, 2000, Vol. 28, No. 8, e35
Non-Patent Document 3: Nucleic Acids Researech, 1990, Vol. 18, No. 17, p.4783

However, in the method of biasing the primer concentrations, it is necessary to optimize the experiment conditions by setting the concentration ratio of the primers, for example.

The method of exploiting the difference in primer amplification ability takes advantage of the fact that RNA primers are not amplified as easily as DNA primers, and thus has the effect of lowering the overall amplification efficiency. It also is extremely expensive to synthesize DNA/RNA primers.

There are instances where the method of utilizing the difference in primer Tm cannot be adopted for the amplification of a desired sequence, because there may not always be a sequence that both corresponds to a sequence to be amplified and that also has a requisite Tm difference.

Methods that use a blocker to inhibit one of the extension reactions lead to an overall drop in PCR efficiency because one of the extension reactions is inhibited. These methods also require arduous tasks such as separately adding a blocker after PCR has begun. Further, the synthetic PNA (peptide nucleic acid) that is used as the blocker is extremely costly.

The present invention was arrived at in order to solve the foregoing issues, and it is an object thereof to provide a DNA amplification method with which it is possible to simply and efficiently prepare a single-strand DNA in a desired direction of a desired region.

SUMMARY OF THE INVENTION

The DNA amplification method of the invention is a DNA amplification method that uses DNA as a template, wherein the DNA amplification method uses a first primer that is complementary to the 3' end side of a target base sequence, a second primer that has a base sequence that is homologous to the 5' end side of that target base sequence, and a third primer that has, on its 3' end side, a base sequence that is homologous to part of the base sequence on the 5' end side of the second primer or the first primer;

wherein a melting temperature $Tm_1$ of the first primer, a melting temperature $Tm_2$ of the second primer, and a melting temperature $Tm_3$ of the third primer have a relationship expressed by the following expression:

$$Tm_1 < Tm_3 \text{ and } Tm_2 < Tm_3$$

and

wherein the DNA amplification method has a first process of performing a PCR cycle at an annealing temperature Ta (where $Ta \leq Tm_1$ and $Ta \leq Tm_2$), and a second process of performing a PCR cycle at an annealing temperature Tb (where $Tm_1 < Tb$, $Tm_2 < Tb$, and $Tb \leq Tm_3$).

It is preferable that the third primer includes, at its 5' end side, a compound selected from the group consisting of LCRed 705, an amino group, a phosphate group, biotin, DIG, DNP, TAMRA, Texas-Red, ROX, XRITC, rhodamine, LCRed 640, a mercapto group, psoralen, cholesterol, FITC, 6-FAM, TET, cy3, cy5, BODIPY 564/570, BODIPY 500/510, BODIPY 530/550, BODIPY 581/591, an oligonucleotide whose total g and c content is 50% or more, and an oligonucleotide of two or more bases whose total g and c content is 15% or more.

It is preferable that the number of PCR cycles in the first process is 10 to 30, and the number of PCR cycles in the second process is 10 to 50.

With the DNA amplification method of the invention, it is possible to simply and efficiently prepare a single-strand DNA of a desired region in a desired direction. Moreover, it is possible to create single-strand DNA with a higher percentage content and absolute amount in the DNA amplification product than has been the case conventionally.

BRIEF DESCRIPTION OF THE DRAWINGS

FIG. 1A to FIG. 1E are a conceptual diagram showing the amplification process according to an embodiment of the invention.

FIG. 2 is a conceptual diagram showing the amplification process according to an embodiment of the invention.

FIG. 3 is a graph showing the fluorescence intensity corresponding to single-strand DNA (ssDNA) and double-strand DNA (dsDNA) in the DNA amplification product obtained in the example.

DETAILED DESCRIPTION OF THE PREFERRED EMBODIMENTS

The DNA amplification method of the invention is a DNA amplification method that uses DNA as a template, and uses a first primer that is complementary to the 3' end side of a target base sequence, a second primer that has a base sequence that is homologous to the 5' end side of the target base sequence, and a third primer that has, at its 3' end side, a base sequence that is homologous to part of the base sequence of the 5' end side of the second primer or the first primer.

The target base sequence (hereinafter, "target sequence") refers to the region to be amplified through the method of the invention, and is a base sequence made of bases arranged in a specific order from the 5' end to the 3' end. In terms of the functionality, etc., of the region to be amplified, this region can be a sense strand or an antisense strand that is complementary to a sense strand.

Hereinafter, "base sequence" may be referred to simply as "sequence."

There are no particular limitations regarding the template for the DNA amplification method of the invention as long as a single-strand DNA can be produced through PCR, and the template may be double-strand DNA or single-strand DNA. Examples of this template (template DNA) include a DNA fraction that has been prepared from a specimen, a double-strand DNA that has been amplified from a DNA fraction in advance through a PCR method that is known to the public, and a cDNA (complementary DNA) obtained from mRNA, for example, that is used as a template for RT-PCR (reverse-transcription PCR).

As discussed later, when the template DNA includes the target sequence and/or base sequence that is complementary to the target sequence, amplification of the target sequence by the invention proceeds.

As for the first through third primers used in this invention, the melting temperature $Tm_1$ of the first primer, the melting temperature $Tm_2$ of the second primer, and the melting temperature $Tm_3$ of the third primer, satisfy the relationship shown in Formula I below.

$$Tm_1 < Tm_3 \text{ and } Tm_2 < Tm_3 \qquad (I)$$

The melting temperature is the temperature at which a double-strand nucleic acid is heat denatured into individual strands. The following measurement methods (a) to (c) are examples of methods for finding the melting temperature of a primer.

(a) Primer absorption of 260 nm wavelength light is measured as the temperature conditions are changed. The point of inflection in the temperature condition - absorption function that is obtained is regarded as the melting temperature Tm of the primer.
(b) A calculated value is used in place of a measured value. For example, the formula and estimated results disclosed on pages 25 to 27 in Special Issue on Cell Technology: Biotechnology Experiments Illustrated 3+ PCR that really amplifies by Hiroki NAKAYAMA and published by Shujunsha, can be used.
(c) After forming double strands of nucleic acid made from a single strand that includes a primer and its complementary strand, an operation of adding a substance that incorporates into the double-strand (a so-called intercalater: for example, ethidium bromide, Cyber Green I) is performed. This operation is performed under various temperature conditions, and the point of inflection in the temperature condition - fluorescence intensity function that is obtained is regarded as the Tm of the primer.

There are no limitations to the invention regarding the method for obtaining the Tm, and it is only necessary that the values for $Tm_1$, $Tm_2$, and $Tm_3$ that are obtained by a common method for the first to third primers satisfy the above Formula (I). It should be noted that the method of (b) illustrated above is simple, and the method of (c) is usually often used. It is preferable that the melting temperature is found under conditions that are identical to the reaction conditions that are actually used in the first process and the second process.

The first primer that is used in the invention is complementary to the 3' end side of the target sequence. In other words, it has at least some of the base sequence that is complementary to a specific base sequence located at the 3' end side of the target sequence. The first primer preferably is made from only a base sequence that is complementary to the target sequence.

The second primer has a base sequence that is homologous to the 5' end side of the target sequence. That is, in at least a portion thereof it includes a base sequence with the same order in the 5' to 3' direction as a specific base sequence located at the 5' end side of the target sequence.

The third primer has, at its 3' end side, a base sequence that is homologous to part of the base sequence of 5' end side of the second primer or the first primer. As discussed later, depending on whether a primer that has a base sequence that is homologous to part of the base sequence of the second primer is used, or whether a primer that has a base sequence that is homologous to part of the base sequence of the first primer is used, it is possible to freely control the direction of the nucleic acid strand that is amplified.

Hereinafter, the sequence homologous to both the third primer and to the second primer or the first primer, and that is located at the 5' end side of the second primer or the first primer and at the 3' end side of the third primer, is called the "common sequence." The common sequence may be a sequence that can be hybridized, or a sequence that cannot be hybridized, to the target sequence or a sequence that is complementary to the target sequence.

Thus, in the present invention, it is possible for the common sequence to be a sequence that cannot hybridize to the target sequence or a sequence that is complementary to the target sequence, and thus preparation of a third primer that contributes to the amplification of a desired target sequence and that satisfies the relationship set forth in Formula I is easy.

The DNA amplification method of the invention is characterized in that it includes a first process of using the first through third primers to perform a PCR cycle at an annealing temperature Ta (where Ta≤$Tm_1$ and Ta≤$Tm_2$), and a second process of using the first through third primers to perform a PCR cycle at an annealing temperature Tb (where $Tm_1$<Tb, $Tm_2$<Tb, and Tb≤$Tm_3$).

Specifically, DNA amplification can be achieved by performing the first process and the second process, which are discussed in detail later, in a PCR reaction solution. The PCR reaction solution includes template DNA, dNTPs (deoxynucleotide triphosphates), DNA polymerase, and a primer set consisting of the first through third primers, and can be prepared by a method that is known to the public. As the DNA polymerase, a thermostable polymerase is preferable, and a Taq polymerase is an illustrative example thereof.

It should be noted that in a case where an inactivated DNA polymerase is used, an activation step for activating the DNA polymerase is performed prior to the first process under conditions that are known to the public.

(First Process)

First, a first process of performing a PCR cycle at an annealing temperature Ta (where Ta≤$Tm_1$ and Ta≤$Tm_2$) is performed.

For example, a PCR cycle (I) consisting of the following steps (1) to (3) is carried out a desired number of times in a PCR reaction solution.

(1) A denaturation step of controlling the temperature to the denaturation temperature of the double-strand DNA.

(2) An annealing step of controlling the temperature to an annealing temperature Ta that satisfies the conditions $Ta \leq Tm_1$ and $Ta \leq Tm_2$.

(3) An extension step of controlling the temperature to a temperature condition that is suited for the DNA extension reaction by the DNA polymerase.

It should be noted that it is also possible to set a temperature condition that is suited for steps (2) and (3) and to perform steps (2) and (3) simultaneously.

(Second Process)

Next, a second process of performing a PCR cycle at an annealing temperature Tb (where $Tm_1 < Tb$, $Tm_2 < Tb$, and $Tb \leq Tm_3$) is performed.

For example, a PCR cycle (II) consisting of the following steps (4) to (6) is carried out a desired number of times.

(4) A denaturation step of controlling the temperature to a desired denaturation temperature.

(5) An annealing step of controlling the temperature to an annealing temperature Tb that satisfies the conditions $Tm_1 < Tb$, $Tm_2 < Tb$, and $Tb \leq Tm_3$.

(6) An extension step of controlling the temperature to a temperature condition that is suited for the DNA extension reaction by the DNA polymerase.

It should be noted that it is also possible to set a temperature condition that is appropriate for steps (5) and (6) and to perform steps (5) and (6) simultaneously.

When the template DNA includes the target sequence and/or a base sequence that is complementary to the target sequence, the target sequence is amplified by performing the PCR cycles (I) and (II).

For example, if a primer that shares a common sequence with the second primer is used as the third primer, then the following reaction takes place.

In a case where the template is double-strand DNA and includes the target sequence, by performing the PCR cycle (I), the first primer is annealed to one of the individual strands of DNA present in the target sequence of the double-strand DNA template. The region of the second primer that is homologous to the 5' end of the target sequence is annealed to the other individual strand DNA, and DNA extension proceeds with the first and second primers serving as origins (5' ends), producing an extended DNA strand whose origin (5' end) is the second primer and an extended DNA strand whose origin is the first primer.

In a case where the template is single-strand DNA and includes the target sequence, by performing the PCR cycle (I), first the first primer is annealed to that single-strand DNA and an extended DNA strand whose origin is the first primer is created. Next, the second primer is annealed to this extended DNA strand and an extended DNA strand whose origin is the second primer and whose end point (3' end) is a sequence complementary to the first primer is created.

In a case where the template is single-strand DNA and includes a base sequence that is complementary to the target sequence, by performing the PCR cycle (I), first the second primer is annealed to that single-strand DNA and an extended DNA strand whose origin is the second primer is created. Next, the first primer is annealed to this extended DNA strand, and an extended DNA strand whose origin is the first primer and whose end point (3' end) is the sequence complementary to the second primer is created.

In a case where a common sequence shared by the second primer and the third primer cannot hybridize to the target sequence or a sequence that is complementary to the target sequence, DNA that can be hybridized with the third primer is not present in the PCR reaction solution at the start of the PCR cycle (I). On the other hand, in a case where the common sequence can hybridize to the target sequence or a sequence that is complementary to the target sequence, DNA to which the third primer can hybridize is present in the PCR reaction solution at the start of the PCR cycle (I).

In all of these cases, repeatedly performing the PCR cycle (I) yields a PCR product that includes an extended DNA strand (I) with the first primer at its 5' end and a sequence that is complementary to the common sequence at its 3' end.

Both the common sequence in the second primer and the common sequence in the third primer can hybridize to the 3' end sequence of the extended DNA strand (I). Consequently, by performing an additional PCR cycle (I) that includes denaturation, annealing, and extension steps after the extended DNA strand (I) has been created, the first through third primers contribute to the DNA extension reaction and result in the accumulation of a large quantity of amplification fragments, from sequences homologous to the first primer (5' end side) to sequences complementary to the common sequence (3' end side).

In the PCR cycle (II), the annealing temperature Tb is set to the above range, and by doing this, neither the first primer nor the second primer can anneal to other nucleotide chains. On the other hand, the third primer anneals to the 3' end side of the amplification fragment, from the sequence that is homologous to the first primer obtained through the first process up to the sequence that is complementary to the common sequence, and then the DNA extension reaction in which dNTPs are added to the 3' end of the annealed third primer takes place.

By repeating this PCR cycle (II), single-strand DNA that is made of a sequence that has the third primer at its 5' end and thereafter is homologous to the target sequence up to its 3' end is accumulated.

The above illustrates an example of the reaction in a case where a primer that shares a common sequence with the second primer is used as the third primer. On the other hand, if a primer that shares a common sequence with the first primer is used as the third primer, then it is possible to create a nucleic acid chain that is complementary to the target sequence mentioned above.

In this way, the first process results in the accumulation of a large quantity of a template that can bind in a complementary manner with the third primer, and then through the second process, only the third primer, of the primers, contributes to the DNA extension reaction, and thus the creation of single-strand DNA whose forward primer is the third primer proceeds extremely efficiently, and single-strand DNA that is constituted by the amplified target sequence is efficiently produced.

After performing the first process and the second process, it is possible to keep the PCR reaction solution at a predetermined temperature. It should be noted that with the present invention, there is little risk that the amplified single-strand DNA will form a double strand even when the PCR reaction solution is kept at a relatively low temperature.

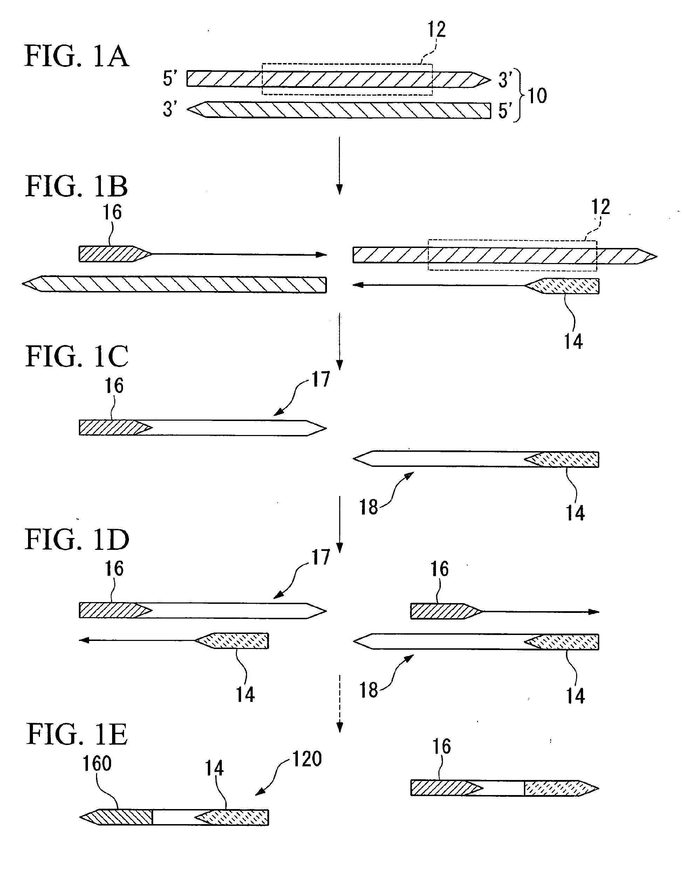

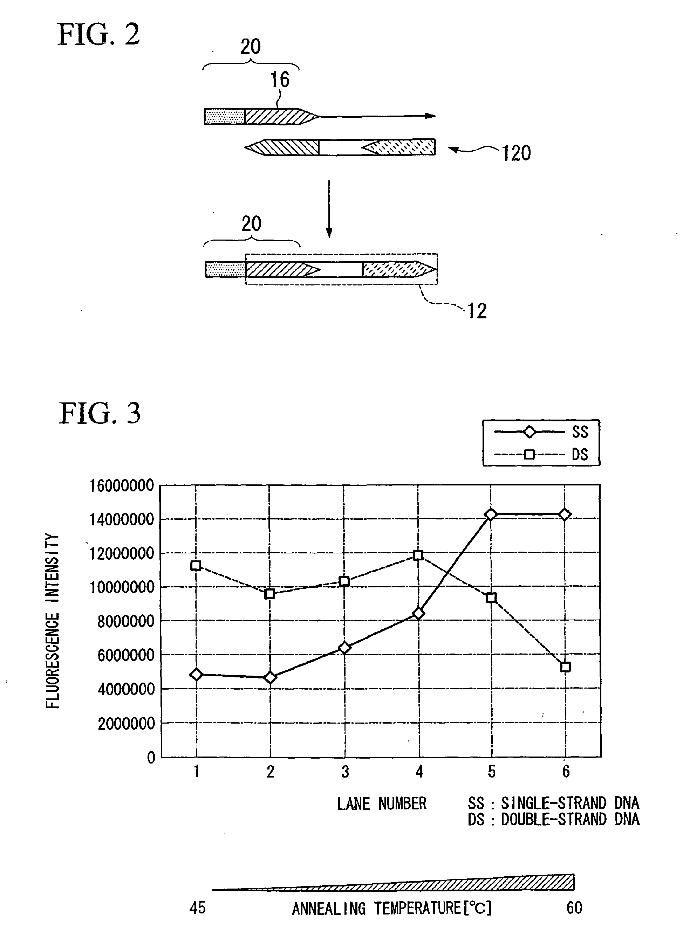

The process of amplifying a target sequence according to an embodiment of the invention is illustrated with reference to FIG. 1A - FIG. 1E, and FIG. 2. This embodiment is an example in which the template is double-strand DNA that includes a target sequence, and a first primer that is complementary to the 3' end side of the target base sequence, a second primer consisting of a base sequence that is homologous to the 5' end side of that target base sequence, and a third primer that at its 3' end side has a common sequence that is homologous to the entire region of the second primer, are used. In FIGS. 1A-1E and 2, the triangular end of the arrow indicates the 5' end of the DNA, and the pointed end indicates the 3' end.

A template 10 shown in FIG. 1A includes a target sequence 12 in one of its individual strands of DNA.

As shown in FIG. 1B, by performing the PCR cycle (I), a first primer 14 is annealed to the one individual strand of DNA, of the two strands of the template DNA, in which the target sequence 12 is present. A second primer 16 is annealed to the other strand of DNA.

Then, DNA extension proceeds with the first and second primers serving as the origins (5' ends) as shown in FIG. 1C, producing an extended DNA strand 17 whose origin (5' end) is the second primer 16 and an extended DNA strand 18 whose origin is the first primer 14.

As shown in FIGS. 1D and 1E, by repeatedly performing the PCR cycle (I), an extended DNA strand 120 that at its 5' end has the first primer 14 and that at its 3' end has a sequence 160 that is complementary to the second primer, is created.

As shown in FIG. 2, by performing the PCR cycle (II), a third primer 20 that in this example has at its 3' end side a common sequence 16 that is homologous to the entire region of the second primer, is annealed to the 3' end side of the extended DNA strand 120, and by repeating the PCR cycle (II), single-strand DNA that has the third primer 20 at its 5' end and that thereafter up to its 3' end is made of the target sequence 12 is accumulated.

(DNA Amplification Product)

Regardless of whether double-strand DNA that includes the target sequence is used as the template, whether single-strand DNA that includes the target sequence is used as the template, or whether single-strand DNA that includes a sequence that is complementary to the target sequence is used as the template, the DNA amplification product that is obtained through the first and second processes includes a large amount of single-strand DNA that is formed by amplifying the target sequence and that corresponds to the combination of the first, second, and third primers.

In the DNA amplification product, the ratio of double-strand DNA and single-strand DNA that have been created can be evaluated by labeling the DNA amplification with fluorescent markers and then measuring the fluorescence intensity of the bands that are separated by polyacrylamide gel electrophoresis. It should be noted that if fluorescent markers are bound to the third primer in advance, the need for this marking operation is obviated, and it is possible to readily confirm that a single-strand DNA that has the desired 5' end side sequence has actually been created.

(First Primer)

The relationship between the melting temperature $Tm_1$ of the first primer and the melting temperature $Tm_2$ of the second primer is the same as that in primer sets used in commonly-used PCR methods, and although there are no particular limitations regarding this relationship, it is preferable that $Tm_1 \approx Tm_2$. Here, $Tm_1 \approx Tm_2$ indicates that the Tm of the first primer and the Tm of the second primer are approximate to the extent that the first primer and the second primer both effectively function as primers when PCR has been performed at a particular annealing temperature.

(Second Primer)

The second primer has, at least in one part thereof, a base sequence that is homologous to the 5' end side of the target sequence.

As mentioned above, a common sequence that is homologous to the 3' end side of the third primer is present at the 5' end side of the second primer or the first primer.

The melting temperatures $Tm_2$ and $Tm_3$ of the second and third primers satisfy the relationship $Tm_2 < Tm_3$. To obtain a second primer that satisfies this relationship, the $Tm_2$ can be adjusted by increasing or decreasing the GC content of the second primer, increasing or decreasing the overall length of the second primer, or through a combination of these methods, for example.

(Third Primer)

The third primer has, at its 3' end side, a common sequence that is homologous to part of the base sequence of the 5' end side of the second primer or the first primer.

With respect to the first and second primers, the third primer satisfies the relationship $Tm_1 < Tm_3$ and $Tm_2 < Tm_3$.

To obtain a third primer that has a higher melting point than the first and second primers in this way, and that exhibits a suitable melting temperature difference with respect to the first and second primers, the $T_3$ can be adjusted by a following method.

A method of increasing or decreasing the GC content of the third primer.

A method of increasing or decreasing the overall length of the third primer.

It is also possible to use a method of controlling the $Tm_3$ by binding a compound that is selected from the specific compound group or the specific bases, which are discussed later, to the 5' end side of the third primer.

It is also possible to combine these methods.

Since the third primer shares a common sequence with the second primer or the first primer used in the invention, increasing or decreasing the length of this common sequence increases or decreases the overall length of both the third primer and the second primer or the first primer. Consequently, in the present invention, increasing and decreasing the length of the common sequence allows the combination of a second primer or a first primer for any target sequence and a third primer that has a desired Tm difference with respect to that second primer or first primer to be obtained with ease, and can be used for the reaction.

It is preferable that at its 5' end side, the third primer includes a compound that is selected from the group consisting of LCRed 705, an amino group, a phosphate group, biotin, DIG, DNP, TAMRA, Texas-Red, ROX, XRITC, rhodamine, LCRed 640, a mercapto group, psoralen, cholesterol, FITC, 6-FAM, TET, cy3, cy5, BODIPY 564/570, BODIPY 500/510, BODIPY 530/550, and BODIPY 581/591 (hereinafter referred to as the "specific compound group"), and an oligonucleotide whose total g and c content is 50% or more and an oligonucleotide of at least two bases whose total g and c content is 15% or more (hereinafter, these are referred to as "specific bases").

Giving the third primer a compound that is selected from the specific compound group or the specific bases, the $Tm_3$ of the third primer can be adjusted with even greater ease. It should be noted that the specific bases preferably are sequences that do not have specificity for the target sequence.

In the list of the specific compound group, DIG is digoxigenin, DNP is dinitrophenyl, TAMRA is carboxytetramethylrhodamine, Texas-Red is 1H, 5H, 11H, 15H-Xantheno[2,3,4-ij:5,6,7-i'j']diquinolizin-18-ium, 9-[2(or 4)-[[[6-(2,5-dioxo-1-pyrrolidinyl)oxy]-6-oxohexyl]amino]sulfonyl]-4(or 2) sulfophenyl]-2,3,6,7,12,13,16,17-octahydro-,inner salt, ROX is rhodamine X, XRITC is rhodamine X isothiocyanate, FITC is fluorescein isothiocyanate, 6-FAM is 6-carboxyfluorescein, TET is tetrachlorofluorescein, BODIPY 564/570 is 4,4-difluoro-5-styryl-4-bora-3a,4a-diaza-s-indacene-3-propionic acid, succinimidyl ester, BODIPY 500/510 is 4,4-difluoro-4-bora-3a,4a-diaza-s-indacene-3,5-dipropionic acid, succinimidyl ester, BODIPY 530/550 is 4,4-difluoro-5,7-diphenyl-4-bora-3a,4a-diaza-s-indacene-3-propionic acid, succinimidyl ester, and BODIPY 581/591 is 4,4-difluoro-5-(4-phenyl-1,3-butadienyl)-4-bora-3a,4a-diaza-s-indacene-3-propionic acid, succinimidyl ester.

A first embodiment of the specific base is an oligonucleotide whose total g and c content (GC content) is 50% or more. A high GC content tends to increase the efficiency of the DNA amplification reaction, and it is possible to set the GC content to 60% or more, 65% or more, 70% or more, 75% or more, 80% or more, 85% or more, 90% or more, 95% or more, and 100%. A GC content of any one of these percentages can be suitably selected by a person skilled in the art by taking into consideration factors such as the length of the specific base itself and being careful that the specific base itself is not complementary to the sequence to be amplified or to the primers. A specific base that is too long may lower the efficiency of the DNA amplification reaction, and thus it is preferable that the specific base is not more than 40 bases long. Further, to reduce the likelihood that primer dimers will form, it is preferable that the specific base includes one of the bases g or c at a percentage of 50% or more or includes one of the bases a or t at a percentage of 50% or more.

A second embodiment of the specific base is an oligonucleotide that has a GC content of 15% or more and that is made of a base sequence of two or more bases. When an oligonucleotide whose GC content is less than 15% is bound, the efficiency of the DNA amplification reaction may drop. It is preferable that the base g or c of the greater content constitutes 70% or more of the combined content of g and c, and that the base a or t of the greater content constitutes 70% or more of the combined content of a and t.

An illustrative example of a specific base sequence preferable as the specific base is a following sequence of two to eight bases. For sequences of 9 or more bases, it is sufficient to suitably combine the following sequences of two to eight bases. It should be noted that S represents c or g, and W represents a or t.

SS, SW, WS, SSS, SSW, SWS, WSS, SSSS, SSSW, SSWS, SWSS, WSSS, SSSSS, SSSSW, SSSWS, SSWSS, SWSSS, WSSSS, SSSSSS, SSSSSW, SSSSWS, SSSWSS, SSWSSS, SWSSSS, WSSSSS, SSSSSSS, SSSSSSW, SSSSSWS, SSSSWSS, SSSWSSS, SSWSSSS, SWSSSSS, WSSSSSS, SSSSSSSS, SSSSSSSW, SSSSSSWS, SSSSSWSS, SSSSWSSS, SSSWSSSS, SSWSSSSS, SWSSSSSS, WSSSSSSS, SSSSSWW, SSSSSWSW, SSSSWSSW, SSSWSSSW, SSWSSSSW, SWSSSSSW, WSSSSSSW, SSSSSWWS, SSSSWSWS, SSSWSSWS, SSWSSSWS, SWSSSSWS, WSSSSSWS, SSSSWWSS, SSSWSWSS, SSWSSWSS, SWSSSWSS, WSSSSWSS, SSSWWSSS, SSWSWSSS, SWSSWSSS, WSSSWSSS, SSWWSSSS, SWSWSSSS, WSSWSSSS, SWWSSSSS, WSWSSSSS, WWSSSSSS.

Another specific example is an oligonucleotide of up to 20 bases that is constituted by a repeating unit of agtc, aagt, ggac, or gggc.

It also is preferable that the specific base has a sequence that forms a secondary structure and does not interfere with the amplification reaction, and more specifically, it is preferable that base pair formation between the sequences is low, and it is particularly preferable that there is no base pair formation between the sequences. At a minimum, it is preferable that consecutive base pairs are not formed. Additionally, it is preferable that base pair formation is low, and particularly preferable that there is no base pair formation, with other sequences in the third primer.

If the compound included in the third primer is a specific base, then it is preferable that a publicly-known radioisotope or fluorescent substance, for example, is bound to the 5' end of the third primer (that is, to the 5' end of the specific base) as a marker substance.

In this invention, by using the third primer in combination with a second primer or a first primer that has a region homologous to the 3' end side of the third primer, it is possible to easily prepare a primer (third primer) that corresponds to the target sequence to be amplified and that has a higher Tm than the first primer and the second primer as a nucleotide that has a common sequence that is homologous to the 5' end side of the second primer. That is, it is easy to provide a third primer that has a $Tm_3$ that satisfies the above Formula I and that preferentially functions in the second process, and also that contributes to amplification of a target sequence that has a desired direction (sequence order from the 5' side to the 3' side). Thus, it is possible to readily provide a primer for a desired sequence and that has a requisite Tm, which was difficult to achieve through conventional so-called thermal asymmetric methods. Further, since it is possible to include a desired compound, such as a specific compound or specific base discussed above, at the 5' end side of the third primer, it is particularly easy to prepare a third primer that has desired properties.

As discussed above, with the DNA amplification method of the invention, the factors that lower the overall PCR efficiency are not necessary, and the first process and the second process contribute to the amplification of single-strand DNA in one direction. Regardless of whether the template is double-strand DNA or single-strand DNA, it is possible to efficiently obtain a single-strand DNA that has a desired direction regardless of the direction of the template DNA, in correspondence with the settings for the first, second, and third primer, and that corresponds to a desired region.

Moreover, it is not necessary to use RNA primers, PNA or other expensive reagents, or special devices. Since the amplification product can be obtained in a single step without requiring an operation for separately adding a blocker, and it is not necessary to asses the concentration ratio of the primers, for example, all the process steps can be finished simply. Consequently, the DNA amplification method of the invention can be practiced simply and inexpensively.

To more efficiently obtain a desired single-strand DNA, it is preferable that the PCR cycle (I) is repeated for 10 to 30 cycles, and that the PCR cycle (II) is repeated for 10 to 50 cycles.

The DNA amplification product yielded by the method of the invention is ideal for analyzing the single nucleotide polymorphisms (SNPs) of genes using single-strand DNA, for example. Consequently, it can be effectively employed to identify microorganisms in the medical, public health, and food sanitation fields, and in gene analysis for clinical diagnoses, for example.

Examples

(Synthesis Example 1) Template DNA

Chromosome DNA extracted from a strain of enteritis vibrio (Serial Number: V89-056, NICHIREI CORPORATION) was used as the template in the examples.

(Synthesis Example 2) Primer

The enteritis vibrio toxin gene (150 base pairs) having the base sequence shown in Sequence Number 1 was taken as the target sequence, and the following primers were synthesized for amplification of that sequence.

First Primer: The base sequence shown in Sequence Number 2. Melting Temperature $Tm_1$=50°C
Second Primer: The base sequence shown in Sequence Number 3. Melting Temperature $Tm_2$=50°C
Third Primer: Primer obtained by binding Cy3 to the oligonucleotide that has the base sequence shown in Sequence Number 4. Melting Temperature $Tm_3$=62°C

(PCR Device)

The PCR Express by Hybaid, furnished with the Gradient Block Module, was used as the PCR device.

(Example 1)

The template DNA obtained in Synthesis Example 1, the first through third primers obtained in Synthesis Example 2, Taq polymerase ("Ampli Taq Gold" made by Applied Biosystems), and the dNTPs that comes with this Taq polymerase ("Amp dNTP MIX") were dissolved in the attached PCR buffer ("Gene Amp 10×PCR Buffer") to produce the PCR reaction solution.
A polymerase activation step was performed in this PCR reaction solution at 95°C for 10 min, and then PCR cycles were performed under the following conditions.
A PCR cycle consisting of (1) denaturation: 95°C, 40 sec; (2) annealing: 45°C, 30 sec; and (3) extension: 72°C, 1 min was repeated for 20 cycles.
A PCR cycle consisting of (4) denaturation: 94°C, 40 sec; (5) annealing: 45°C to 60°C at six gradients (lanes 1 to 6 of the gradient block module), 30 sec; and (6), extension: 72°C, 1 min was repeated for 40 cycles.
Next, the reaction product was held at 72°C for 10 minutes and then cooled to 4°C, and the DNA amplification product was recovered.

(Evaluation Method)

The amplification product that was obtained was analyzed by polyacrylamide gel electrophoresis. The conditions for this analysis were as follows.

Migration condition gel concentration: 5%; Migration
conditions: 1×TBE, 100 V; Analysis condition device used:
Fluoro Image Analyzer ("FLA-8000" by Fuji Photo Film Co., Ltd.), detection wavelength: 532 nm

The fluorescence intensities at the detection wavelength 532 nm corresponding to ssDNA (single-strand DNA) and dsDNA (double-strand DNA) are shown in FIG. 3.
In Example 1, single-strand DNA was obtained in large quantities. In particular, the amount of single-strand DNA that was obtained under the condition of performing PCR for 20 cycles at an annealing temperature of 45°C and then increasing the annealing temperature by approximately 10°C or more and performing PCR for 40 cycles (lanes 5 and 6) was significantly greater than the amount of double-strand DNA (mass ratio with respect to dsDNA: approximately 1.5 to 3).

(Comparative Example 1)

The same method as that of Example 1 was performed except that the third primer was not used, and the DNA amplification product was analyzed.
The amplification product was separated by polyacrylamide gel electrophoresis and subjected to silver staining, and it was found that although bands corresponding to the size of double-strand DNA were detected, bands corresponding to single-strand DNA were not detected for any annealing temperature condition.

INDUSTRIAL APPLICABILITY

With the DNA amplification method of the invention, it is possible to simply and efficiently prepare a single-strand DNA of a desired region in a desired direction. Moreover, it is possible to create single-strand DNA with a higher percentage content and absolute amount in the DNA amplification product than has been the case conventionally.

**Claims**

1. A DNA amplification method that uses DNA as a template, **characterized in that**:

the DNA amplification method uses a first primer that is complementary to the 3' end side of a target base sequence, a second primer that has a base sequence that is homologous to the 5' end side of that target base sequence, and a third primer that has, on its 3' end side, a base sequence that is homologous to part of the base sequence on the 5' end side of the second primer or the first primer; a melting temperature $Tm_1$ of the first primer, a melting temperature $Tm_2$ of the second primer, and a melting temperature $Tm_3$ of the third primer have a relationship expressed by the following expression:

$$Tm_1 < Tm_3 \text{ and } Tm_2 < Tm_3$$

and
the DNA amplification method comprises a first process of performing a PCR cycle at an annealing temperature Ta (where $Ta \leq Tm_1$ and $Ta \leq Tm_2$), and a second process of performing a PCR cycle at an annealing temperature Tb (where $Tm_1 < Tb$, $Tm_2 < Tb$, and $Tb \leq Tm_3$).

2. The DNA amplification method according to claim 1, wherein the third primer comprises, at its 5' end side, a compound selected from the group consisting of LCRed 705, an amino group, a phosphate group, biotin, DIG, DNP, TAMRA, Texas-Red, ROX, XRITC, rhodamine, LCRed 640, a mercapto group, psoralen, cholesterol, FITC, 6-FAM, TET, cy3, cy5, BODIPY 564/570, BODIPY 500/510, BODIPY 530/550, BODIPY 581/591, an oligonucleotide whose total g and c content is 50% or more, and an oligonucleotide of two or more bases whose total g and c content is 15% or more.

3. The DNA amplification method according to claim 1 or 2, wherein the number of PCR cycles in the first process is 10 to 30, and the number of PCR cycles in the second process is 10 to 50.

FIG. 1A

FIG. 1B

FIG. 1C

FIG. 1D

FIG. 1E

## FIG. 2

## FIG. 3

LANE NUMBER          SS : SINGLE-STRAND DNA
                     DS : DOUBLE-STRAND DNA

45          ANNEALING TEMPERATURE [°C]          60

## INTERNATIONAL SEARCH REPORT

| International application No. |
| --- |
| PCT/JP2005/009885 |

**A. CLASSIFICATION OF SUBJECT MATTER**
Int.Cl$^7$  C12N15/09

According to International Patent Classification (IPC) or to both national classification and IPC

**B. FIELDS SEARCHED**

Minimum documentation searched (classification system followed by classification symbols)
Int.Cl$^7$  C12N15/00-15/90, C12Q1/68

Documentation searched other than minimum documentation to the extent that such documents are included in the fields searched

Electronic data base consulted during the international search (name of data base and, where practicable, search terms used)
BIOSIS/MEDLINE/WPIDS(STN), JSTPlus(JOIS)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2003-199568 A  (Nichirei Corp.), 15 July, 2003 (15.07.03), & US 2004/110182 A1 | 1-3 |
| A | WO 2003/054233 A1  (Brandeis University), 03 July, 2003 (03.07.03), & JP 2005-512577 A     & EP 1468114 A1 & US 2004/053254 A1 | 1-3 |
| A | Sanchez J.A. et al., Linear-after-the-exponential (LATE)-PCR: an advanced method of asymmetric PCR and its uses in quantitative real-time analysis, Proc.Natl.Acad.Sci.USA, 17 February, 2004 (17.02.04), 101(7), p.1933-8 | 1-3 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 10 June, 2005 (10.06.05) | 28 June, 2005 (28.06.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/009885 |

C (Continuation).    DOCUMENTS CONSIDERED TO BE RELEVANT

| Category* | Citation of document, with indication, where appropriate, of the relevant passages | Relevant to claim No. |
| --- | --- | --- |
| P,A | JP 2004-337129 A  (Canon Inc.),<br>02 December, 2004 (02.12.04),<br>(Family: none) | 1-3 |
| P,A | JP 2005-162 A  (Canon Inc.),<br>06 January, 2005 (06.01.05),<br>(Family: none) | 1-3 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5849497 A

- US 5627054 A

### Non-patent literature cited in the description

- *Proceedings of the National Academy of Sciences,* 1988, vol. 85, 7652-7656
- *Nucleic Acids Researech,* 2000, vol. 28 (8), e35
- *Nucleic Acids Researech,* 1990, vol. 18 (17), 4783

- **HIROKI NAKAYAMA.** Special Issue on Cell Technology: Biotechnology Experiments Illustrated 3+ PCR that really amplifies. Shujunsha, 25-27